# EUROPÄISCHE PATENTANMELDUNG

(11) **EP 3 311 860 A1**
(43) Veröffentlichungstag der Anmeldung: **25.04.2018**
(21) Anmeldenummer: 16194899.7
(22) Anmeldetag: 20.10.2016
(51) Int. Cl.: A61M 1/10

(54) **KANÜLE, KANÜLENSYSTEM, HERZPUMPENSYSTEM UND VERFAHREN ZUR VOLUMENENTLASTUNG EINES HERZENS**

(71) Anmelder: Berlin Heart GmbH, 12247 Berlin (DE)
(72) Erfinder: MENON, Ares K., 12209 Berlin (DE); GRÄBNER, Franziska, 10625 Berlin (DE)
(74) Vertreter: Pfenning, Meinig & Partner mbB

(57) **Zusammenfassung**

Die Anmeldung betrifft eine Kanüle zur Entlastung des linksseitigen Herzens mit einem Kanülenschaft, welcher einen herzseitigen Einlass und einen pumpenseitigen Auslass umfasst. Zwischen dem Einlass und dem Auslass erstreckt sich ein Lumen, wobei an einer Außenseite des Kanülenschafts ein Nahtring zur Verbindung der Kanüle mit einem linken Atrium angeordnet ist. Der Auslass ist derart konfiguriert, dass der Auslass mit einer Pumpe verbindbar ist und der Kanülenschaft zwischen dem Nahtring und dem Auslass eine derartige Länge besitzt, dass der Kanülenschaft durch einen Interkostalraum nach außen führbar ist.

## Beschreibung

Gegenstand der vorliegenden Anmeldung ist eine Kanüle zur Entlastung des Herzens, ein Kanülensystem, das eine derartige Kanüle sowie einen Obturator oder einen Trokar umfasst, ein Herzpumpensystem, das eine Herzpumpe sowie zwei Kanülen umfasst, und weiterhin ein Verfahren zur Volumenentlastung eines Herzens.

Bei akutem Linksherzversagen nach Myokardinfarkt, Dekompensation einer Herzinsuffizienz oder bei anderen Pathologien der Linksherzfunktion mit Dekompensation entsteht eine Pumpschwäche der linken Herzkammer, die unter anderem drei Effekte hat. Erstens treten eine Unterversorgung des Körperkreislaufs mit Sauerstoff und nährstoffreichem Blut, eine Übersäuerung des Gewebes (Azidose) sowie ein drohendes Organversagen auf. Zweitens kommt es zu einer Dilatation des linken Ventrikels mit erhöhter Volumen- und Druckbelastung. Drittens treten ein Blutrückstau in der Lungenstrombahn mit erhöhtem pulmonalem Kapillardruck sowie Lungenhochdruck und ein drohendes Lungenödem auf.

Beim Auftreten des Linksherzversagens werden oftmals intra-aortale Ballonpumpen (IABP), aber auch oftmals extrakorporale Herzunterstützungssysteme für eine extrakorporale Membranoxigenation (ECMO) eingesetzt. Der Zugang zu großen Körpergefäßen wird dabei zumeist über die Leistengefäße hergestellt. Innerhalb weniger Minuten kann so ein suffizienter Kreislauf im Patienten sichergestellt werden. Da bei einem akuten Linksherzversagen häufig eine Lungenstauung mit Lungenödem vorliegt, wird ein Oxigenator zur verbesserten Sauerstoffzufuhr in Serie geschaltet. Jedoch ist beim Linksherzversagen das mangelnde Pumpverhalten des linken Ventrikels ursächlich, die schlechte Oxigenierung ist nur sekundär.

Dennoch hat sich auch bei der Patientenkohorte mit primärem Linksherzversagen die ECMO durchgesetzt, weil sie schnell und effektiv den marginalen oder fehlenden Kreislauf wiederherstellt. Daher wurde der Begriff der extrakorporalen Lebensunterstützung (extracorporal life support, ECLS) eingeführt, um diese Patientenkohorte von der reinen ECMO-Lungen-Versagerkohorte abzugrenzen. Bei der ECMO wird eine große Körpervene als Zustrom zur Pumpe bzw. zum Oxigenator und eine große Arterie als Einstrom des Blutes in den Körperkreislauf gewählt. Aufgrund dieser Kanülierungstechnik kommt es nicht zu einer direkten Volumenentlastung des schwer kontraktionsgestörten linken Ventrikels. Somit ist eine eventuelle Erholung der linksventrikulären Kontraktilität kaum möglich.

Weitere beschriebene Verfahren zur Entlastung des linken Ventrikels, z. B. über den linken Vorhof mittels eines Katheters oder eine Atrio-Septektomie, können keine effiziente Entlastung des linksventrikulären Cavums erzielen. Daher sind die Chancen, dass sich ein Patient allein durch die ECLS in seiner linksventrikulären Kontraktilität erholt, sehr gering.

Eine effektive Möglichkeit bieten jedoch linksventrikuläre Unterstützungssysteme, sogenannte "left-ventricular assist devices" (LVAD). Diese sind jedoch in der Implantationstechnik komplexer und kosten ein Vielfaches der oben genannten ECMO, IABP oder ECLS Pumpensysteme.

Mit der vorliegenden Erfindung soll ein effizientes, kostenoptimiertes System zur rasch anwendbaren und sicheren Implantation am Herzen geschaffen werden, so dass die Kontraktilität des linken Ventrikels verbessert werden kann.

Die Aufgabe wird mithilfe der in den Ansprüchen genannten Kanülen, des Kanülensystems und des Herzunterstützungssystems gelöst. Ferner wird ein Verfahren zur Volumenentlastung des linksseitigen Herzens skizziert.

In einer ersten Ausführungsform umfasst die Kanüle zur Entlastung des linksseitigen Herzens einen Kanülenschaft mit einem herzseitigen Einlass und einem pumpenseitigen Auslass. Zwischen dem Ein- und dem Auslass erstreckt sich ein Lumen, durch welches das Blut von der linken Herzseite, vorzugsweise dem Atrium oder dem Ventrikel, aus dem Körper herausgeführt werden kann. Der Auslass ist dabei so konfiguriert, dass er mit einer Pumpe verbindbar ist.

An einer Außenseite des Kanülenschafts ist ein Nahtring befestigt, mit dem die Kanüle an einer Wand des linken Atriums befestigt werden kann. Hierzu kann beispielsweise eine minimalinvasive rechtsseitige Thoraktomie mit anschließender Inzision des Herzbeutels durchgeführt werden, wobei das linke Atrium freigelegt wird, Hochnähte an der Herzwand verlegt und anschließend durch den Nahtring gezogen werden.

In dieser Ausführungsform besitzt die Kanüle eine Wandstärke entlang des Kanülenschafts, welche ein formstabiles Lumen zulässt. Dies bedeutet, dass bereits vergleichsweise hohe Kräfte angelegt werden müssen, um das Lumen abzuklemmen, und die im Blutkreislauf vorherrschenden Drücke nicht ausreichen, um das Lumen zu kollabieren.

Wie bereits eingangs erwähnt, ist die Kanüle so ausgebildet, dass sie vom linken Atrium durch einen Interkostalraum auf einer von vorne betrachtet rechten Seite eines Patienten nach außen geführt werden kann (in anderen Ausführungsbeispielen kann die Kanüle auch auf der anderen, linke Seite ausgeführt werden). Durch eine derartige Länge wird gewährleistet, dass der Patient nach dem Anschluss der Kanüle an eine Herzpumpe nicht bettlägerig sein muss, da kein Kanülenauslass durch die Femoralarterie geführt wird. Von daher erlangt der Patient schneller seine Mobilität wieder, so dass der Gesundungsprozess besser unterstützt wird.

Um eine anmeldungsgemäße Kanüle zu implantieren, wird, wie bereits erwähnt, das linke Atrium mittels einer minimalinvasiven rechtsseitigen Thoraktomie freigelegt, und der Herzbeutel mit Hochnähen versehen. Die Kanüle wird beispielsweise zunächst mit einem Trokar versehen, der das Lumen der Kanüle blockiert. Beispielsweise nach Heparingabe wird die Atriumswand mittels einer Seldinger-Technik punktiert und anschließend ein Führungsdraht vorgeschoben, entlang welchem ein Trokar eingebracht wird, über den die Kanüle in den linken Vorhof bzw. in den linken Ventrikel vorgeschoben wird. Anschließend wird der Nahtring der Kanüle mit der Wand des linken Atriums verbunden, so dass die Kanüle fest an der Herzwand anliegt; dadurch, dass die Kanüle vorzugsweise durch einen Interkostalraum geführt wird, wirken jedoch nur geringe Kräfte auf die Wand des Vorhofes. Anschließend wird der Auslass der Kanüle mit dem Einlass einer extrakorporalen Herzpumpe oder einem VAD verbunden.

In einer Ausführungsform der Kanüle weist diese einen Kanülenschaft mit einer Länge von mehr als 20 cm, vorzugsweise von mehr als 30 cm auf. Auf diese Weise wird gewährleistet, dass die Kanüle so weit aus dem Brustkorb ragen kann, dass der Anschluss einer extrakorporalen Herzpumpe problemlos möglich ist und die Mobilität des Patienten gewährleistet wird.

In einer weiteren Ausführungsform besitzt der Kanülenschaft eine Wandstärke von 2 bis 4 mm. Eine derartige Wandstärke gewährleistet, dass die Kanüle bzw. das Lumen der Kanüle unter den im Blutkreislauf vorherrschenden Druckverhältnissen nicht kollabiert und nicht merklich in seinem Querschnitt eingeschränkt wird. Hierdurch wird ein stabiler Blutfluss durch die Kanüle gewährleistet.

In einer weiteren Ausführungsform ist der Kanülenschaft aus einem biokompatiblen Material, wie beispielsweise Silikon, gefertigt. Biokompatible Materialien eignen sich bevorzugt für eine temporären Implantation und anschließende Explantation, sobald der Patient nicht mehr auf eine externe Herzunterstützung angewiesen ist oder sobald klar ist, dass der Patient dauerhaft ein LVAD benötigt. Vorzugsweise ist der Kanülenschaft an seiner Außenseite derart glatt, dass er nur schwer mit dem Gewebe verwachsen kann. Hierdurch wird gewährleistet, dass auch nach mehreren Wochen Implantationszeit die Kanüle problemfrei explantiert werden kann.

In einer weiteren Ausführungsform umfasst die Kanüle eine Druckmessleitung mit einem Druckeinlass und einem Druckauslass, wobei der Druckeinlass der Druckmessleitung herzseitig des Nahtrings angeordnet ist. Dies bedeutet, dass der Druckeinlass im Wesentlichen zwischen dem Nahtring und dem (Kanülen-) Einlass angeordnet ist. Dabei ist der Querschnitt der Druckmessleitung kleiner als der Querschnitt des Kanülenlumens. Vorzugsweise ist der Querschnitt der Druckmessleitung mindestens zehnmal kleiner als der Querschnitt des Lumens der Kanüle. Auf diese Weise wird gewährleistet, dass die Druckmessleitung den Durchmesser der Kanüle nur unwesentlich erhöht. Mittels der Druckmessleitung ist es möglich, den Druck innerhalb des linken Atriums dauerhaft zu überwachen, ohne eine gesonderte Druckmessleitung beispielsweise über einen Port zeitweise in die Kanüle einschieben zu müssen.

Vorzugsweise ist die Druckmessleitung an einer Außenseite des Kanülenschafts entlang geführt. Dabei kann die Druckmessleitung beispielsweise mit der Außenseite des Kanülenschafts verschweißt oder verklebt sein.

In einer weiteren speziellen Ausführungsform ist die Druckmessleitung im Lumen der Kanüle geführt.

In einer weiteren speziellen Ausführungsform ist die Druckmessleitung als gesondertes Lumen in der Kanülenschaftwand angeordnet.

Die Druckmessleitung ist so konfiguriert, dass sie mit einem externen Druckmesssystem, beispielsweise einem handelsüblichen Blutdruckmesssystem wie den Intensivmonitoren von Philips, HP oder Siemens, verbindbar ist. Hierbei ist der Druckauslass beispielsweise mit einem Adapter ausgestattet, der normiert ist und mit dem gewählten System gekoppelt werden kann. Obgleich in zahlreichen Ausführungsformen die Druckmessleitung mit der Außenseite des Kanülenschafts stoffschlüssig verbunden ist, kann die Druckmessleitung auslassseitig über den Auslass der Kanüle hinausragen und deutlich länger als der Kanülenschaft sein, so dass eine Kopplung an ein externes Druckmesssystem möglich ist.

Alternativ zur oder in Kombination mit der Druckmessleitung kann in der Nähe des Einlasses der Kanüle ein Drucksensor, wie beispielsweise ein mechanisch-elektromagnetischer Sensor, angeordnet sein. Auch ein mit einer Membran versehener Drucksensor kann am Einlass der Kanüle angeordnet werden.

In einer weiteren Ausführungsform umfasst der Einlass mindestens einen Drainagekorb. Unter einem Drainagekorb wird eine Anzahl von Öffnungen verstanden, die die Wand des Kanülenschafts umlaufend in der Nähe des Einlasses angeordnet sind. Auch eine einzige dort angebrachte Öffnung soll von diesem Begriff hier umfasst sein. Mittels des Drainagekorbs, der beispielsweise eine, vorzugsweise zwei, drei, vier oder mehr Öffnungen umfassen kann, wird gewährleistet, dass Blut nicht nur durch den Einlass allein, sondern auch im Wesentlichen senkrecht zu diesem durch die Löcher des Drainagekorbs in das Lumen des Kanülenschafts gesogen werden kann. Auf diese Weise werden größere Totvolumina im Atrium vermieden und die Gefahr einer Thrombenbildung gesenkt. Zudem verbessern sich die Strömungseigenschaften innerhalb des Atriums und beim Einsaugen des Blutes.

In einer weiteren Ausführungsform sind zwischen dem Nahtring und dem Einlass mindestens zwei, voneinander beabstandete Drainagekörbe angeordnet.

Dabei kann in einer weiteren Ausführungsform ein erster Drainagekorb so angeordnet sein, dass er im linken Atrium angeordnet ist, während der zweite Drainagekorb sowie der Einlass durch die Mitralklappe in den linken Ventrikel ragen und hier Blut ansaugen können. Mittels mehrerer in verschiedenen linksseitigen Teilen des Herzens angeordneter Drainagekörbe wird eine besonders gute Volumenentlastung des linken Atriums und des linken Ventrikels erreicht. Dabei ist der Kanülenschaft so ausgebildet, dass er zwischen dem Nahtring und dem Einlass eine solche Länge besitzt, dass der Einlass durch das linke Atrium in den linken Ventrikel ragt.

In einer weiteren Ausführungsform umfasst die Kanüle zwischen dem Nahtring und dem Einlass ein kontrastgebendes Material, wie beispielsweise ein röntgenkontrastgebendes Material, wie beispielsweise ein Metall, oder ein bei einer Echokardiographie kontrastgebendes Material. Auf diese Weise können bei einer minimalinvasiven Implantation mittels einer Röntgenkontrastvorrichtung oder eines Echokardiographen die Positionen des Einlasses und des Nahtrings im Kreislaufsystem des Patienten überwacht werden.

Ferner kann die Kanüle Teil eines Kanülensystems sein, welches neben der Kanüle einen Obturator oder einen Trokar umfasst. Dabei sind der Trokar bzw. der Obturator so ausgebildet, dass sie das Lumen der Kanüle vollständig blockieren. Unter einem Trokar wird hierbei eine Vorrichtung verstanden, die zum Punktieren der Atriumwand bzw. Vergrößern einer bestehenden Punktierung in der Atriumwand während eines Vorgehens nach der Seldinger-Technik verwendet werden kann. Eine Obturator hat keine solche Spitze, sondern dient ausschließlich zur Blockierung des Lumens, so dass die Kanüle ex- bzw. implantiert werden kann.

Vorzugsweise kommt eine in dieser Anmeldung beschriebene Kanüle in einem Herzpumpensystem zur Anwendung, das neben der Kanüle eine extrakorporale Herzpumpe oder ein VAD sowie eine weitere Kanüle aufweist. Die extrakorporale Herzpumpe kann eine beliebige zur extrakorporalen Blutversorgung eines Patienten zugelassene Herzpumpe sein. Der Auslass der hier beschriebenen Kanüle wird mit dem Einlass der Pumpe verbunden, und der Auslass der Pumpe kann über einen Oxigenator oder ohne das Vorhandensein eines Oxigenators mit dem Einlass der weiteren Kanüle verbunden werden. Dabei ist die weitere Kanüle so beschaffen, dass sie beispielsweise mit einer Schlüsselbeinarterie oder einer Leistenarterie verbindbar ist. Hierbei wird das Verbinden mit einer Schlüsselbeinarterie bevorzugt, da auf diese Weise die Mobilität des Patienten weniger eingeschränkt ist und somit eine weitere Verbesserung des Zustands des Patienten erreicht werden kann.

Bei einem Verfahrens zur Implantation bzw, zur Volumenentlastung des linksseitigen Herzens wird die Kanüle in einigen Ausführungsformen zwischen zwei Rippen in den Interkostalraum geschoben und vor der Inbetriebnahme der Pumpe ein Abstandshalter zwischen den Rippen angeordnet, so dass bei Bewegungen des Patienten die sich aufeinander zu bewegenden Rippen die dazwischen liegende Kanüle nicht abklemmen können und es so zu einer Unterversorgung des Patienten mit Blut kommt.

Weitere Details und Ausführungsformen werden anhand der nachfolgenden Figuren näher erläutert. Es zeigen:
Fig. 1 Schematische Ansicht eines ventralen Schnitts durch ein Herz
Fig. 2 Ausführungsform einer Kanüle;
Fig. 3 weitere Ansicht der Kanüle der Fig. 2;
Fig. 4 schematische Ansicht einer implantierten Kanüle;
Fig. 5 weitere Ausführungsform einer Kanüle;
Fig. 6 weitere Ausführungsform einer Kanüle;
Fig. 7a bis 7c Ausführungsform einer Anordnung einer Druckmessleitung;
Fig. 8 weitere Ausführungsform einer Kanüle;
Fig. 9 weitere Ausführungsform einer Kanüle;
Fig. 10 weitere Ausführungsform einer Kanüle;
Fig. 11 bis 13 schematische Ansichten einer implantierten Kanüle;
Fig. 14 beispielhafte Darstellung eines Herzpumpensystems.

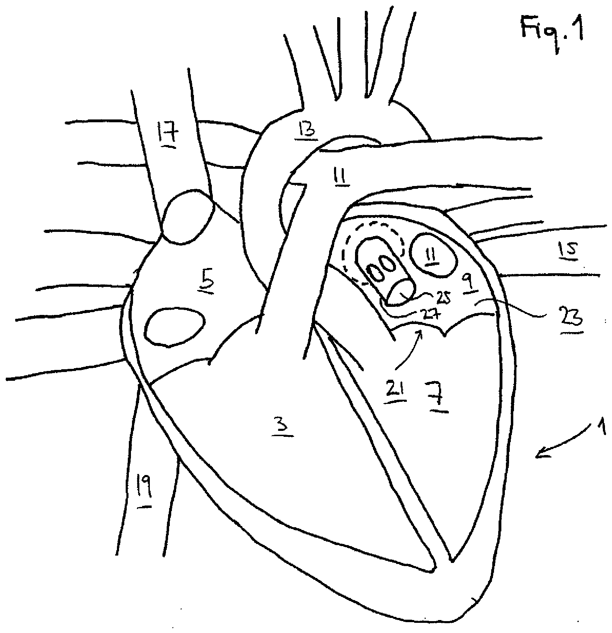

In der Fig. 1 ist ein menschliches Herz 1 in einem ventralen Schnitt dargestellt. Zu sehen ist der rechte Ventrikel 3, sowie das rechte Atrium 5, das linke Ventrikel 7 und das linke Atrium 9. Ferner sind die Pulmonalarterie 11, die Aorta 13, die Pulmonalvenen 15, als auch die Superior Vena Cava 17 und die Inferior Vena Cava 19 zu sehen. Das linke Atrium 9 erhält Blut aus den Pulmonalvenen 15. Dieses fließt normalerweise durch die Mitralklappe 21 in das linke Ventrikel 7 und von dort durch die Aorta 13 in den Körper. Bei einer linken Herzinsuffizienz reicht die Pumpleistung des linken Ventrikels 7 nicht mehr aus, um den Körper ausreichend mit Blut zu versorgen. Aus diesem Grund schlägt die vorliegende Anmeldung vor, eine Kanüle durch die hintere Atriumswand 23 zu führen, wie dies in der Fig. 1 durch den Einlass 25 einer schematischen Kanüle 27 dargestellt wird. Zu den Einzelheiten einer derartigen Kanüle wird in den nachfolgenden Figuren näher Stellung bezogen.

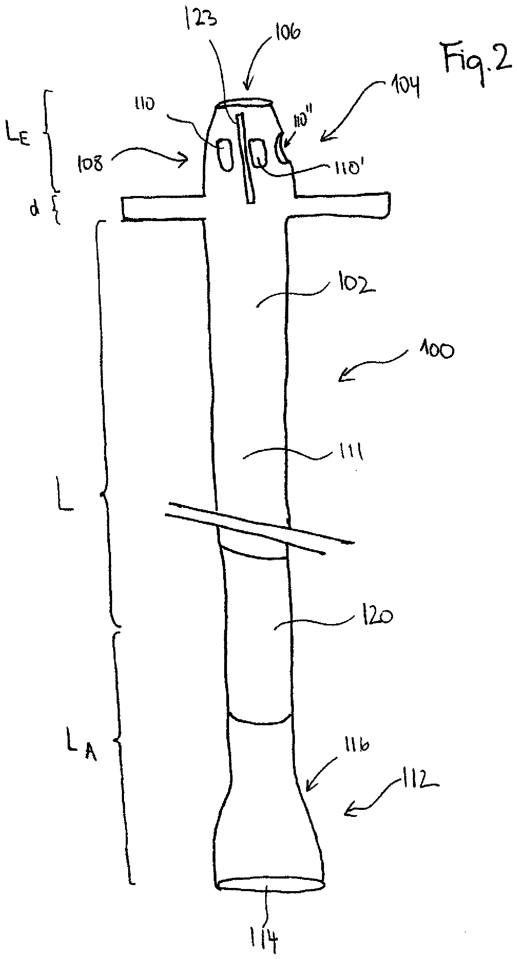

In der Fig. 2 ist eine exemplarische Kanüle 100 dargestellt. Die Kanüle umfasst einen Kanülenschaft 102, welcher beispielsweise aus Silikon bestehen kann. Silikon hat den Vorteil, dass es ein biokompatibles Material ist und vom menschlichen Körper nicht abgestoßen wird. Jedoch können auch andere biokompatible Kunststoffe als Material oder Materialkombination verwendet werden. Beispielhafte Materialien sind Polyurethane.110 Die Oberfläche des Kanülenschaftes 102 hat vorzugsweise eine geringe Rauigkeit, d.h. besitzt vorzugsweise keine Strukturierung der Außenwand des Kanülenschafts, so dass ein Einwachsen mit dem Gewebe, durch welches der Kanülenschaft geführt wird, vermieden wird. Nachfolgend werden die Begriffe "distal" und "proximal" zur Orientierung einzelner Komponenten der Kanüle eingeführt. Dabei bezeichnet "distal" eine Orientierung vom Chirurgen weg und "proximal" eine Orientierung zu diesem hin.

Am distalen Ende des Kanülenschafts 102 befindet sich ein Einlass 104. Der Einlass 104 umfasst eine distale Öffnung 106, sowie einen Drainagekorb 108 mit Drainagekorböffnungen 110, 100' und 110". Der Einlass ist derart konfiguriert, dass dieser im linken Atrium positioniert wird bzw. positionierbar ist. D.h., dass die Kanüle in verschiedenen Größen herstellbar ist, um mit unterschiedlich großen Herzen verbunden zu werden. Durch die distale Öffnung 106 kann Blut in ein Lumen 111 des Kanülenschaftes 102 gesogen werden.

Am proximalen Ende des Kanülenschaftes befindet sich ein Auslass 112 mit einer proximalen Öffnung 114. Der Auslass 112 umfasst einen aufgeweiteten Bereich 116, welcher einen größeren Durchmesser als ein Großteil des übrigen Kanülenschafts 102 besitzt. Dieser aufgeweitete Bereich 116 kann beispielsweise aufgeweitet sein, um eine Verbindung mit einer Pumpe eines Herzpumpensystems herstellen zu können. In anderen Ausführungsformen einer Kanüle kann diese am Auslass einen Adapter zum Anschluß an eine Pumpe aufweisen. Diese Adapter können genormt sein, und beispielsweise einen Durchmesser von 3/8 oder ½ Zoll besitzen.

Auf einem begrenzten Abschnitt des Kanülenschafts 102 ist ein künstliches Gewebe 120 angeordnet, welches das Einwachsen mit einem Körpergewebe begünstigt. Beispielsweise kann es sich bei dem Gewebe um einen Filz handeln, welcher aus einem biokompatiblen Material besteht. Dieses Gewebe ist vorzugsweise in einem Bereich des Kanülenschafts 102 angeordnet, in welchem die Kanüle 100 durch die Haut eines Patienten tritt. Aufgrund des Verwachsens des Gewebes 120 mit dem Körpergewebe im Bereich des Durchtritts der Kanüle durch die Haut wird das Infektionsrisiko für einen Patienten verringert.

In der Nähe des Einlasses 104 befindet sich weiterhin ein Nahtring 122, welcher den Kanülenschaft 102 vorzugsweise vollständig umläuft. Dieser Nahtring besitzt einen größeren Durchmesser als das Lumen 111 und dient einer Verbindung der Kanüle mit der Außenseite der hinteren Atriumswand. Der Nahtring kann beispielsweise aus Silikon bestehen oder dieses umfassen, umfasst jedoch vorzugsweise ein vernähbares textiles Material, da dieses auf einfache Art und Weise mit der Atriumswand vernäht werden kann. Da der Nahtring auf der Außenseite des Atriums verbleibt, befindet sich lediglich der Einlass 104 im Atrium, sobald die Kanüle mit dem Herzen vernäht wurde. Die Länge L_{E} des Einlasses entlang einer Längsachse des Kanülenschafts beträgt je nach Ausführungsform zwischen 0,5 und 3 cm. Die Dicke d des Nahtringes entlang der Längsachse der Kanüle ist vorzugsweise weniger als 1 mm, maximal 0,5 cm. Zwischen dem Nahtring und dem Gewebe 120 erstreckt sich die Kanüle über eine Länge L. Diese Länge L beträgt vorzugsweise mindestens 20 bzw. 30 cm oder länger. Die Länge L sollte dabei derart beschaffen sein, dass die Kanüle von der Atriumswand durch einen Interkostalraum nach Außen geführt werden kann und eine ausreichende Länge L_{A} außerhalb des Körpers verbleibt, so dass die Kanüle mit einem Herzpumpensystem verbunden werden kann und die Länge L_{A} eine ausreichende Mobilität des Patienten erlaubt. In einigen Ausführungsformen sind hier Längen L_{A} von mehr als 30 cm, beispielsweise zwischen 20 und 140 cm, vorzugsweise zwischen 20 und 110 cm umfasst. Die Länge L_{A} kann auch derart gewählt werden, dass ein Patient die extrakorporale Blutpumpe in einer Tasche am Körper tragen kann. Dabei kann ein Großteil der Länge L_{A}, welche sich an das Gewebe anschließt außerhalb des Körpers verbleiben, beispielsweise kann es sich bei der Länge L_{A} um eine Länge zwischen 20 und 80 cm handeln. Als Gesamtlänge L_{E}, L, und L_{A} können sich so je nach Größe und Alter des Patienten Längen zwischen 50 cm und 180 cm ergeben.

Wie bereits erwähnt, umfasst der Einlass 104 eine distale Öffnung 106 und einen Drainagekorb 108. Der Drainagekorb 108 ist derart beschaffen, dass dieser die Strömungsverhältnisse innerhalb des Lumens 111 verbessert. Die distale Öffnung 106 kann beispielsweise einen Durchmesser quer zur Längsachse der Kanüle von 0,3 cm bis 3 cm, vorzugsweise von 0,5 cm bis 1,5 cm, umfassen. Die distale Öffnung 106 kann eine runde oder ovale Grundfläche besitzen und ist vorzugsweise von einem distalen Ende einer Drainagekorböffnung 110 beispielsweise zwischen 0,2 cm und 1 cm beabstandet. Die Größe einer Drainagekorböffnung 110 kann beispielsweise 25 mm² betragen, kann jedoch größer oder kleiner sein. Insgesamt kann ein Drainagekorb 108 mehr als eine Drainagekorböffnung 110, vorzugsweise zwei oder mehr, besonders bevorzugt vier oder mehr Drainagekorböffnungen umfassen. Die Drainagekorböffnungen sind in einigen Ausführungsformen kleiner als die distale Öffnung und liegen proximal dieser. Jedoch befinden sich sämtliche Drainagekorböffnungen distal des Nahtringes, so dass diese bei einer Implantation der Kanüle auch sämtlich im Atrium liegen.

Der Einlass 104 umfasst ferner einen kontrastgebenden Streifen 123, welcher beispielsweise aus einem Metall gefertigt und in das Silikonmaterial des Einlasses eingelassen ist. Mithilfe des kontrastgebenden Streifens kann beispielsweise bei der Implantation der Kanüle im Rahmen einer Fluoreszenzspektroskopie kontrolliert werden, ob sich die Kanüle an der gewünschten Stelle im Atrium befindet.

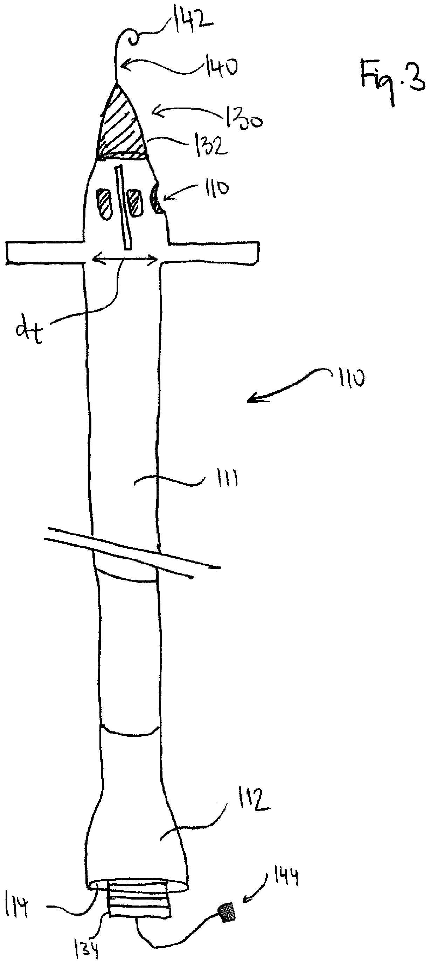

In der Fig. 3 ist die Kanüle 100 in Kombination mit einem Trokar 130 dargestellt. Der Trokar 130 wird in das Lumen 111 eingeführt und verschließt bzw. blockiert dieses. Der Trokar ist länger als die Kanüle und reicht so über die distale Öffnung 106 und über die proximale Öffnung 114 hinaus. Der Trokar dient bei der Implantation dazu, die Kanüle an und in die Atriumswand zu führen.

Die Kanüle kann beispielsweise mithilfe der Seldinger-Technik eingesetzt werden. Hierzu wird zunächst ein Führungsdraht (guidewire) 140 durch den Interkostalraum zwischen zwei Rippen bis zur hinteren Atriumswand geschoben. Das distale Ende 142 des Führungsdrahtes wird dabei unter anderem zur Punktion des Herzens eingesetzt. Anschließend wird das distale Ende 132 des Trokars 130 auf das proximale Ende 144 des Führungsdrahtes gefädelt (der Trokar besitzt hierfür ein für den Führungsdraht 140 geeignetes Lumen) und der Trokar zusammen mit der Kanüle, wie in der Fig. 3 gezeigt, bis zur hinteren Atriumswand geschoben. Der Trokar wird anschließend zur Punktierung der Atriumswand eingesetzt, so dass dieser zusammen mit dem Einlass 104 der Kanüle 100 in das Atrium geschoben wird. Anschließend kann der Nahtring 122 fest mit der hinteren Atriumswand vernäht werden und nachdem der Nahtring 122 fest mit der hinteren Atriumswand verbunden ist, kann der Trokar 130 aus dem Lumen 111 der Kanüle 100 herausgezogen werden und der Auslass 112 der Kanüle 100 mit dem Herzpumpensystem verbunden werden. Beim Vernähen kann auch auf Hochnahttechnicken zurückgegriffen werden und der Nahtring ist dann dementsprechend ausgelegt.

Der Durchmesser dₜ des Trokars 130 entspricht dem Durchmesser des Lumens 111, so dass das Lumen 111 fluiddicht verschlossen ist. Insbesondere kann auch durch die Drainagekorböffnungen 110 keine Flüssigkeit durch das Lumen 111 zur proximalen Öffnung 114 dringen, solange der Trokar im Lumen der Kanüle 100 angeordnet ist.

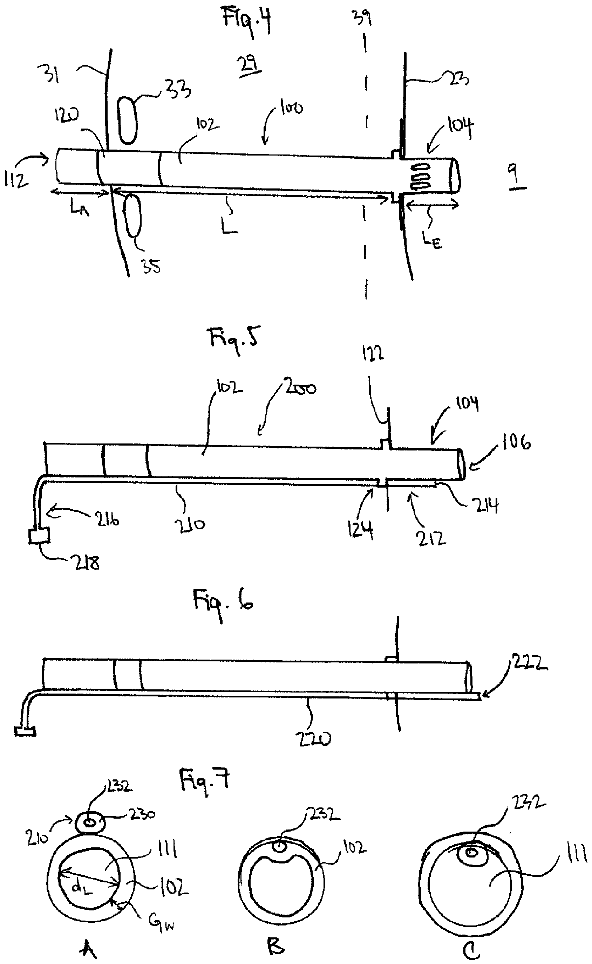

Anhand der Fig. 4 wird schematisch dargestellt, wie eine implantierte Kanüle 100 im Körper liegt. Die Kanüle 100 entspricht dabei beispielsweise einer der in den Fig. 2 und 3 dargestellten Kanülen. Der Einlass 104 ragt in das linke Atrium 9, wobei der Nahtring 122 mit der hinteren Atriumswand 23 vernäht ist. Alternativ oder zusätzlich kann der Nahtring mit der Atriumswand verklebt werden. Der Kanülenschaft 102 erstreckt sich über die Länge L hinweg von der hinteren Atriumswand 23 durch den Interstitialraum 29, durch den Interkostalraum zwischen den Rippen 33 und 35 und durch die Haut 31. Das Gewebe 120 ist derart auf dem Kanülenschaft angeordnet, dass dieses im Bereich des Durchstoßes 37 durch die Haut 31 zum Liegen kommt und so ein Verwachsen des Gewebes mit der Haut 31 begünstigt, so dass das Infektionsrisiko für den Patienten minimiert wird. Außerhalb des Körpers kommt derjenige Teil des Kanülenschaftes 102 mit der Länge L_{A} zum Liegen. Der Auslass 112 wird wiederum beispielsweise mit einem Herzpumpensystem verbunden. Die Länge L_{A} kann beispielsweise zwischen 20 und 80 cm betragen. In weiteren Alternativen sind auch größere Längen als 80 cm, beispielsweise 140 cm, möglich. Zur besseren Orientierung hinsichtlich der Führung der Kanüle 100 wurde noch die Körperachse 39 eingezeichnet. Anhand dieser soll verdeutlicht werden, dass die Kanüle von der hinteren Atriumswand 23 durch die, aus ventraler Sicht, linken Rippenbögen geführt wird.

Obgleich bislang lediglich beschrieben wurde, dass der Einlass, insbesondere dessen distale Öffnung, im Atrium zum Liegen kommt, kann die Länge L_{E} auch derart gewählt werden, dass die Distale Öffnung im linken Ventrikel zum Liegen kommt. Falls ein Drainagekorb vorhanden ist, können die Drainagekorböffnungen derart angeordnet sein, dass diese bei einer implantierten Kanüle entweder im Ventrikel oder im Atrium liegen kommen. Die Abstände der distalen Öffnung und des Drainagekorbs werden dann entsprechend abgestimmt. In anderen Ausführungsbeispielen kann die Kanüle anstatt einer distalen Öffnung ausschließlich Drainagekorböffnungen umfassen.

Anhand der Fig. 5 soll eine weitere Variante einer Kanüle beschrieben werden. Die in der Fig. 5 dargestellte Kanüle 200 umfasst im Wesentlichen sämtliche Merkmale der Kanüle 100, jedoch zusätzlich noch eine Druckmessleitung 210, welche sich entlang des Kanülenschaftes 102 erstreckt. Die Druckmessleitung 210 besitzt einen Druckmessleitungseinlass 212 mit einer distalen Öffnung 214, welcher proximal der distalen Öffnung 106, jedoch distal des distalen Endes einer Drainagekorböffnung 110 liegt. Der Druckmessleitungsauslass 216 liegt proximal der proximalen Öffnung 114 der Kanüle 110 und umfasst einen Adapter 218, welcher zum Anschluss an ein externes Druckmesssystem ausgebildet ist. Beispielsweise kann es sich bei dem Anschluss um einen Snapfit-Connector oder einen Luerverschluss zum Anschluss an ein externes Druckmesssystem handeln. Derartige Adapter sind im Stand der Technik hinlänglich bekannt. Mittels der Druckmessleitung 210, weiche in die Kanüle 200 integriert ist, wird erreicht, dass zu jedem Zeitpunkt der Druck im Atrium gemessen werden kann. Hierdurch entfällt das Einfädeln einer zusätzlichen Druckmessleitung in die Kanüle selbst, welche in regelmäßigen oder unregelmäßigen Abständen durchgeführt werden muss, um die Druckverhältnisse im Atrium zu ermitteln. Im vorliegenden Beispiel der Fig. 5 verläuft die Druckmessleitung 210 außerhalb des Kanülenschaftes 102 und ist an diesem stoffschlüssig befestigt. Dabei kann die Druckmessleitung 210 kann mit der Außenwand des Kanülenschaftes 102 entweder verklebt oder verschweißt sein. Die Druckmessleitung 210 wird dabei durch eine Öffnung 124 oder einen Ausschnitt des Nahtrings 122 geführt, so dass sichergestellt wird, dass der Druckmessleitungseinlass 212 innerhalb des Atriums zum Liegen kommt. Obgleich im vorliegenden Ausführungsbeispiel die distale Öffnung 214 des Druckmessleitungseinlasses 212 proximal der distalen Öffnung 106 liegt, kann, wie in der Fig. 6 gezeigt, in einem anderen Ausführungsbeispiel eine distale Öffnung 224 eines Druckmessleitungseinlasses 222 distal der distalen Öffnung 106 der Kanüle 100 liegen. In anderen Ausführungsbeispielen kann die distale Öffnung des Druckmessleitungseinlasses bündig mit der distalen Öffnung 106 der Kanüle 100 abschließen.

Anhand der Figuren 7A bis 7C sollen die verschiedenen Positionierungen der Druckmessleitung im Querschnitt dargestellt werden. In der Fig. 7A ist die Wand des Kanülenschafts 102 mit der Wanddicke G_{w} dargestellt. Die Wanddicke hat vorzugsweise eine gleichbleibende Dicke zwischen 1 bis 5 mm. Das Lumen 111 der Kanüle 100 besitzt einen Durchmesser D_{L} von 0,3 bis 2 cm. Die Druckmessleitung 210 ist außerhalb des Lumens 111 angeordnet und weist eine separate Leitungswand 230 auf. Die Leitungswand kann dabei eine vorzugsweise konstante Wandstärke zwischen 0,5 bis 3 mm besitzen. Die Flüssigkeit, das heißt im vorliegenden Beispiel Blut, kann durch die distale Öffnung in das Lumen 232 eintreten, bis zum Adapter gelangen und so einer Druckmessung mittels eines Druckmessers im externen Druckmesssystem zugeführt werden. Alternativ kann innerhalb der Druckmessleitung ein Druckmesssensor angeordnet werden. Hierbei kann es sich beispielsweise um eine in der Druckmessleitung 210 angeordnete Membran handeln, wobei die auf die Membran wirkende Kraft in einen Druck umgerechnet werden kann. Hierzu muss die Auslenkung bzw. die auf die Membran wirkende Kraft an ein externes Auswertesystem weitergereich werden. Das Weiterreichen kann beispielsweise mittels einer elektrischen Leitung, die innerhalb der Wand 230 verläuft, vorgenommen werden. Um jedoch eine hohe Flexibilität zu gewährleisten, wird die eigentliche Druckmessung oftmals im externen Druckmesssystem stattfinden.

In der Fig. 7B ist das Lumen 232 in die Wand des Kanülenschafts 102 integriert. In der Fig. 7C ist die Druckmessleitung (ähnlich wie in der Fig. 7A) separat, das heißt nicht in der Wand des Kanülenschafts 102, angeordnet, verläuft jedoch innerhalb des Lumens 111. Auch hier kann die Druckmessleitung stoffschlüssig mit der Wand des Kanülenschafts 102 verbunden sein.

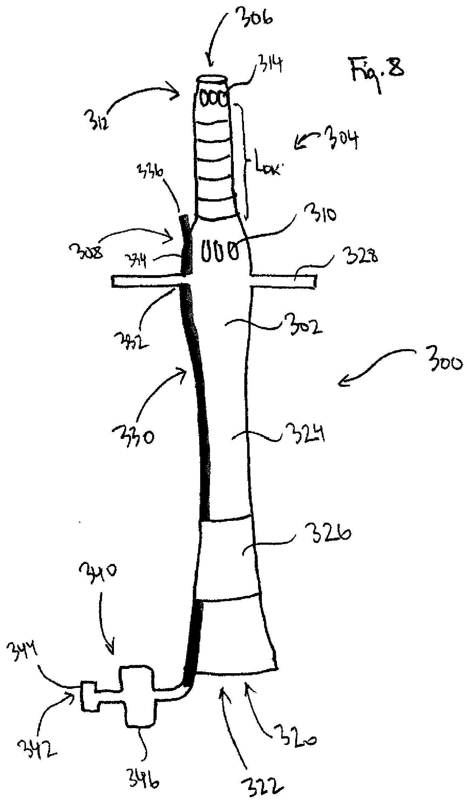

Anhand der Fig. 8 soll eine weitere Ausführungsform einer Kanüle zur Entlastung des Herzens erläutert werden. Die Kanüle 300 umfasst (ähnlich wie die Kanüle 100) einen Kanülenschaft 302, welcher aus Silikon gefertigt ist. Ferner umfasst die Kanüle einen Einlass 304, welcher neben der distalen Öffnung 306 einen ersten Drainagekorb 308 mit einer Vielzahl von Öffnungen 310, sowie einen distal des ersten Drainagekorbs 308 gelegenen zweiten Drainagekorb 312 umfasst, welcher eine Vielzahl von Öffnungen 314 besitzt. Der erste Drainagekorb 308 und der zweite Drainagekorb 312 sind eine Distanz L_{dk} voneinander entfernt. Diese Länge L_{dk} wird derart gewählt, dass der erste Drainagekorb im Atrium gelegen ist, während der zweite Drainagekorb 312 und die distale Öffnung 306 sich durch die Mitralklappe hindurch in den linken Ventrikel erstrecken. Dies hat den Vorteil, dass Blut sowohl aus dem Ventrikel in das Lumen des Kanülenschafts 302 gesogen werden kann und ferner durch die Öffnungen 310 auch Blut aus dem Atrium in den Kanülenschaft gesogen werden kann.

Optional befinden sich zwischen dem ersten und dem zweiten Drainagekorb 308 bzw. 312 kontrastgebende Streifen 316, welche quer zur Längsachse des Kanülenschafts angeordnet sind. Diese kontrastgebenden Streifen 316 bilden eine Alternative zu dem kontrastgebenden Streifen 123, wie dies in der Fig. 2 beschrieben war. Ferner können die kontrastgebenden Streifen aus Draht bestehen und somit dem Bereich zwischen dem ersten und dem zweiten Drainagekorb eine zusätzliche Stabilität verleihen. Alternativ können die Streifen lediglich eine zusätzliche Stabilisierung der Kanüle bewirken.

Der distalen Öffnung 306 gegenüber gelegen befindet sich der Auslass 320 mit einer proximalen Öffnung 322. Zwischen der proximalen Öffnung 322 und der distalen Öffnung 306 erstreckt sich das Lumen 324, welches ferner über die Öffnungen 314 und 310 zugänglich ist.

Ferner umfasst die Kanüle 300 eine Filzschicht 326, welche sich über einen Bereich von ca. 10 cm, vorzugsweise weniger als 10 cm, jedoch mehr als 5 cm, erstreckt. Dem Filz 326 kommt dabei die gleiche Aufgabe zu, wie dem Gewebe 120 in der Fig. 2. Insbesondere bedeutet dies, dass sich zwischen dem Nahtring 328 und dem Filz 326 die Länge des Kanülenschafts derart bemisst, dass dieser von der hinteren Atriumswand bis zur Haut im Bereich des Hautdurchstoßes reicht. Vom Filz 326 bis zum Auslass 320 erstreckt sich der Kanülenschaft nochmals beispielsweise zwischen 50 und 80 cm, wobei der Auslass 320 an ein Herzpumpensystem angeschlossen wird.

Optional kann die Kanüle 300 eine Druckmessleitung 330 umfassen, wie diese beispielsweise anhand der Fig. 5 bis 7 bereits erläutert wurde. Die Druckmessleitung 330 ist derart beschaffen, dass diese durch eine Öffnung 332 im Nahtring reicht und dort einen Einlass 334 bildet. Der Einlass besitzt eine distale Öffnung 336, welche distal des distalen Endes der Öffnungen 310 liegt. Die Druckmessleitung ist dabei derart beschaffen, dass diese den Druck im Atrium messen kann. An ihrem proximalen Ende befindet sich ein Auslass 340, welcher einen Adapter 342 mit einer proximalen Öffnung 344 umfasst, weicher beispielsweise an ein externes Druckmesssystem angeschlossen werden kann. Zusätzlich besitzt die Druckmessleitung 330 einen Drucksensor 346 mit einer eigenen Auswerteeinrichtung, so dass der Atriumsdruck zum einen über ein externes Druckmesssystem jedoch auch durch den intrinsischen Drucksensor 346 gemessen werden kann.

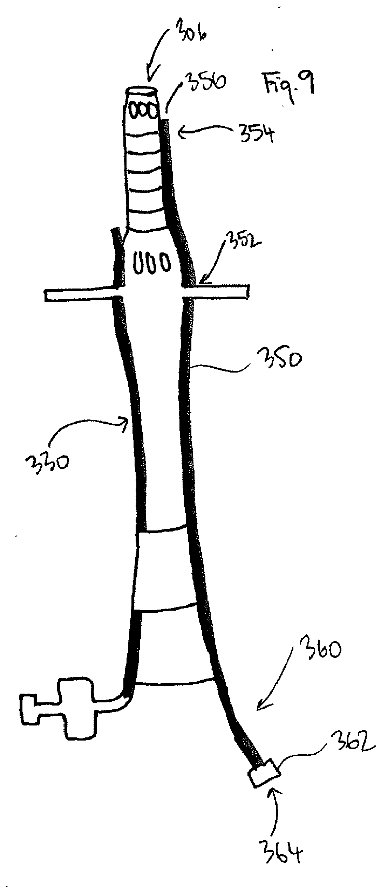

In einer weiteren Alternative umfasst die Kanüle 300, wie in der Fig. 9 gezeigt, eine zweite Druckmessleitung 350. Diese kann optional oder alternativ zu der Druckmessleitung 330 vorhanden sein. Die zweite Druckmessleitung 350 wird durch eine weitere Öffnung 352 durch den Nahtring geführt und deren Einlass 354 kommt mit seiner distalen Öffnung 356 proximal des proximalen Endes der Öffnungen des zweiten Drainagekorbs 314 zum Liegen. Dabei ist die distale Öffnung 356 jedoch derart angeordnet, dass diese ebenfalls innerhalb des Ventrikels zum Liegen kommt. Die zweite Druckmessleitung 350 besitzt ferner einen Auslass 360 mit einem Adapter 362 über dessen proximale Öffnung 364 die zweite Druckmessleitung 350 mit einem externen Druckmesssystem verbunden werden kann. Da die zweite Druckmessleitung im Ventrikel endet, kann ebenfalls zu jedem Zeitpunkt und ohne weiteren chirurgischen Eingriff der Druck im Ventrikel gemessen werden.

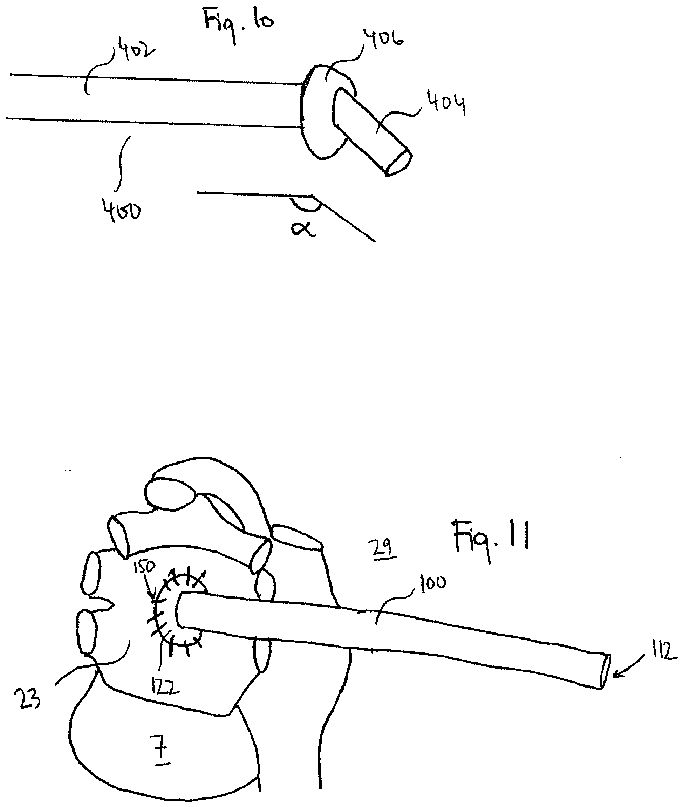

In einer alternativen Ausführungsform können die bisher dargestellten Ausführungsformen der Kanüle einen Winkel zwischen dem Einlass und dem verbleibenden Kanülenschaft aufweisen. Dies wird anhand der Fig. 10 illustriert. Die Kanüle 400 umfasst einen Kanülenschaft 402, einen Einlass 404 sowie einen Nahtring 406. Zwischen dem Einlass 404 und dem Schaft 402 befindet sich eine Biegung um einen Winkel α, welcher zwischen 30° und 75° betragen kann. Dabei ist im Bereich der Biegung vorzugsweise proximal des Nahtringes 406 angeordnet und trägt zur Form der Kanüle bei, d.h. ohne dem Einwirken weiterer Kräfte zeigt die Kanüle diese Biegung. Durch das Einführen eines Trokars, beispielsweise des Trokars wie in der Fig. 3 gezeigt, kann die Biegung auf einen Winkel von weniger als 5° begradigt werden, was das Einführen der Kanüle in den Körper bis zur hinteren Atriumswand erleichtern kann. In anderen Alternativen kann der Trokar auch derart ausgestaltet sein, dass dieser zunächst gerade eingeführt werden kann, so dass die Kanüle 400 gestreckt vorliegt, der Trokar jedoch über einen Drahtmechanismus, ähnlich einem Drahtmechanismus eines steuerbaren Katheters, beim Erreichen der hinteren Atriumswand um den Winkel α gebogen wird, erst anschließend ein Durchstoßen der hinteren Atriumswand mittels des Trokars vorgenommen wird und erst dann der Einlass 404 in das Atrium eingeführt wird. Ein derartiger Trokar mit einer Drahtsteuerung zum Verbiegen kann unabhängig von den hier genannten Kanülen beansprucht werden.

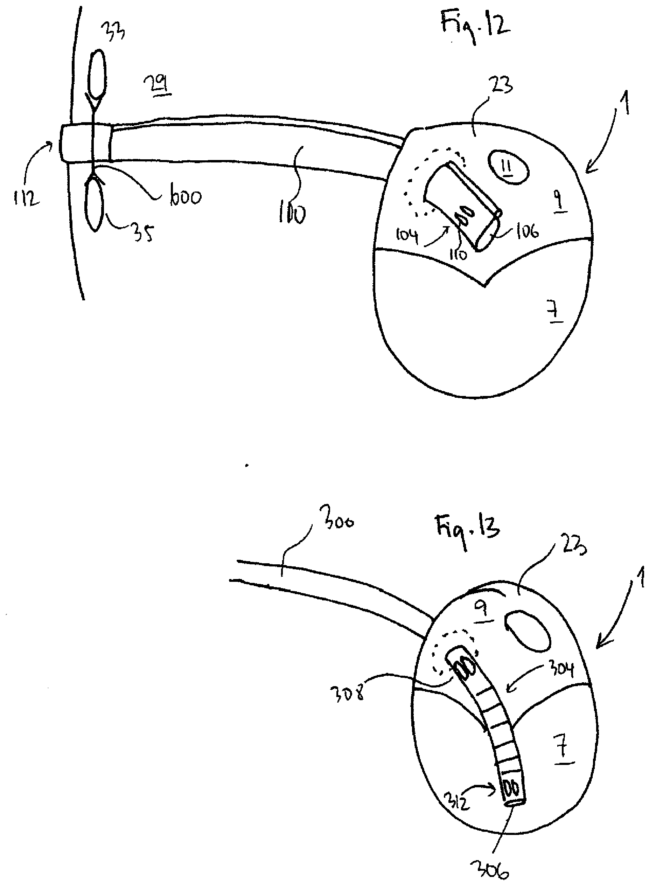

Die Fig. 11 zeigt einen schematischen, dorsalen Schnitt durch den Brustkorb eines Menschen, so dass eine implantierte Kanüle 100 sichtbar ist. Die Kanüle 100 erstreckt sich von rechts nach links durch den Interstitialraum und wird durch eine in dieser Aufsicht nicht sichtbare Öffnung in der hinteren Atriumswand 23 in das linke Atrium (und je nach Ausführungsform ggf. bis ins Ventrikel) geführt. Der Nahtring 122 ist mittels einer Naht oder einer Vielzahl von Nähten 150 mit der hinteren Atriumswand verbunden. Betrachtet man den in der Fig. 11 gezeigten Ausschnitt in einer ventralen Ansicht, ergibt sich die in der Fig. 12 schematisch dargestellte Situation. Es ist deutlich erkennbar, dass die Kanüle 100 durch den Interstitialraum 29 und durch die hintere Atriumswand 23 in das linke Atrium 9 geführt wurde. Auf diese Weise erstreckt sich der Einlass 104 in das Atrium (oder in anderen Ausführungsformen der Kanüle in das Ventrikel). Durch die distale Öffnung 106 und die Drainagekorböffnungen 110 kann nun Blut mittels einer am Auslass 112 angeordneten Herzpumpe aus dem Atrium (bzw. Ventrikel) in die Pumpe gesaugt werden und anschließend durch einen Pumpenauslass bzw. eine mit dem Pumpenauslass verbundene Kanüle beispielsweise der Subclavia zugeführt werden. Um beispielsweise ein Verschließen der Kanüle 100 im Interkostalraum zwischen den Rippen 33 bzw. 35 zu vermeiden, kann zwischen den beiden Rippen ein entsprechender Abstandshalter, beispielsweise aus Metall oder Plastik eingefügt werden. Dieser Abstandshalter 600 verhindert, dass die beiden Rippen 33 bzw. 35 derart aufeinandergedrückt werden können, dass das Lumen 111 der Kanüle 100 versperrt wird und kein Blut mehr durch die Kanüle 100 gefördert werden kann.

In der Fig. 13 ist die Situation dargestellt, welche nach der Implantation einer Kanüle 300 vorliegt. Der Einlass 304 führt von der hinteren Atriumswand 23 in das linke Atrium 9 und den linken Ventrikel 7. Dabei liegen die distale Öffnung 306, sowie der zweite Drainagekorb 312 innerhalb des Ventrikels und der erste Drainagekorb 308 innerhalb des Atriums. Auf diese Weise kann durch eine am Auslass der Kanüle 300 angeordnete Pumpe Blut sowohl aus dem Atrium als auch dem Ventrikel gesogen werden und beispielsweise über eine weitere Kanüle der (Arteria) Subclavia zugeführt werden. Obgleich in den Fig. 12 und 13 keine Druckmessleitungen dargestellt sind, können diese optional wie in den vorhergehenden Figuren beschrieben, Teil der Kanüle sein.

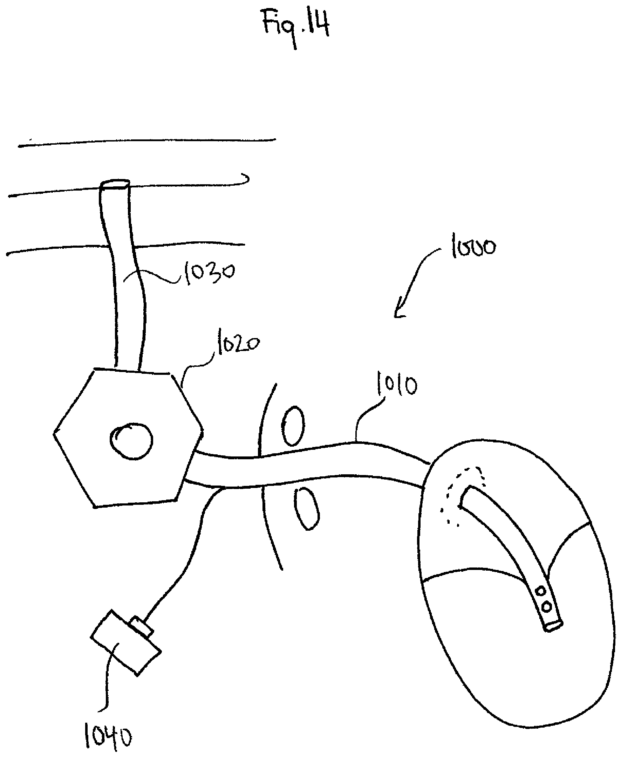

Anhand der Fig. 14 soll kurz beschrieben werden, wie ein anmeldungsgemäßes Herzpumpensystem mit einer der vorab beschriebenen Kanülen konfiguriert sein kann. Das in der Fig. 14 dargestellte System 1000 umfasst neben einer Kanüle 1010, welche beispielsweise durch eine der in den Fig. 2 bis 13 beschriebenen Kanülen oder eine andere sich aus den Ausführungen dieser Anmeldung ergebenen Kanülen gegeben sein kann, eine Blutpumpe 1020, welche beispielsweise eine Herzpumpe für eine ECMO, ECLS oder andere Herzunterstützungstätigkeit ausgebildet sein kann. Die Herzpumpe ist außerhalb des menschlichen Körpers angeordnet und wird mit dem Auslass der Kanüle 1010 verbunden. Die Herzpumpe 1020 kann auch eine Kurzzeitpumpe sein, welche gemäß den Regularien lediglich für wenige Tage oder Monate bei einem Menschen eingesetzt werden kann. Bei längerer Notwendigkeit einer herzunterstützenden Pumpentätigkeit kann diese Pumpe einfach ersetzt werden, ohne, dass der Patient einer weiteren Operation unterzogen werden muss. Mögliche Pumpen zur Verwendung im Herzunterstützungssystem sind beispielsweise Centrimag^{®} oder Pedimag^{®} von Thoratec, Rotaflow^{®} oder Rotassist^{®} von Maquet, DP3 von Medos, Revolution^{®} von Sorin, Biomedicus^{®} von Medtronic oder ähnliche auf dem Markt erhältliche Pumpen.

Der Ausgang der Pumpe kann über eine weitere Kanüle beispielsweise mit einer Subclavia des Menschen verbunden werden, so dass die Blutpumpe mit den Kanülen 1010 und 1030 einen geschlossenen Kreislauf bildet. Eine Druckmessleitung der Kanüle 1010 kann über einen entsprechenden Adapter mit einem externen Druckmesssystem 1040 verbunden werden. Externe Druckmesssysteme sind im klinischen Bereich hinlänglich bekannt und ausreichend vorhanden. Über eine Anzeige des Druckmessgerätes 1040 kann somit jederzeit der arterielle bzw. ventrikuläre Druck während der Versorgung des Patienten mit dem Herzunterstützungssystem 1000 eingesehen werden.

## Patentansprüche

1. Kanüle zur Entlastung des linksseitigen Herzens, umfassend einen Kanülenschaft mit einem herzseitigen Einlass und einem pumpenseitigen Auslass und einem sich zwischen dem Einlass und dem Auslass erstreckenden Lumen, wobei an einer Aussenseite des Kanülenschafts ein Nahtring zur Verbindung der Kanüle mit einem linken Atrium angeordnet ist und der Auslass derart konfiguriert ist, dass der Auslass mit einer Pumpe verbindbar ist und der Kanülenschaft zwischen dem Nahtring und dem Auslass eine derartige Länge besitzt, dass der Kanülenschaft durch einen Interkostalraum nach Aussen führbar ist.

2. Kanüle nach Anspruch 1, wobei ferner eine Druckmessleitung mit einem Einlass und einem Auslass vorhanden ist, wobei der Einlass des Druckmesssystems herzseitig des Nahtrings angeordnet ist.

3. Kanüle nach Anspruch 2, wobei die Druckmessleitung an der Aussenseite des Kanülenschafts entlang geführt ist.

4. Kanüle nach Anspruch 2, wobei die Druckmessleitung im Lumen geführt ist.

5. Kanüle nach einem der Ansprüche 2 bis 4, wobei der Auslass der Druckmessleitung derart konfiguriert ist, dass diese mit einem externen Druckmesssystem koppelbar ist.

6. Kanüle nach einem der vorhergehenden Ansprüche, wobei der Kanülenschaft eine Länge von mehr als 20 cm, vorzugsweise von mehr als 30 cm besitzt.

7. Kanüle nach einem der vorhergehenden Ansprüche, wobei der Kanülenschaft eine Wandstärke zwischen 1 mm und 5 mm besitzt.

8. Kanüle nach einem der vorhergehenden Ansprüche, wobei der Kanülenschaft aus einem biokompatiblen Material, vorzugsweise Silikon, gefertigt ist.

9. Kanüle nach einem der vorhergehenden Ansprüche, wobei diese einen Drucksensor umfasst.

10. Kanüle nach einem der vorhergehenden Ansprüche, wobei der Einlass mindestens einen Drainagekorb umfasst.

11. Kanüle nach einem der vorhergehenden Ansprüche, wobei der Kanülenschaft zwischen dem Nahtring und dem Einlass eine derartige Länge besitzt, dass der Einlass durch das linke Atrium in den linken Ventrikel ragt.

12. Kanüle nach den beiden vorhergehenden Ansprüchen, wobei zwischen dem Nahtring und dem Einlass ein erster und ein zweiter Drainagekorb derart angeordnet und voneinander beabstandet sind, so dass der erste Drainagekorb im linken Atrium und der zweite Drainagekorb im linken Ventrikel zum Liegen kommt.

13. Kanüle nach einem der vorhergehenden Ansprüche, wobei zwischen dem Nahtring und dem Einlass ein kontrastgebendes Material vorhanden ist.

14. Kanülensystem, umfassend eine Kanüle nach einem der vorhergehenden Ansprüche und einen das Lumen versperrenden Obturator oder Trocar.

15. Herzpumpensystem, umfassend eine extrakorporale Herzpumpe, eine Kanüle nach einem der Ansprüche 1 bis 13, sowie einer weiteren Kanüle, wobei der Auslass des Kanülenschafts mit einem Einlass der Pumpe und ein Einlass der weiteren Kanüle mit einem Auslass der Pumpe verbunden sind, und die weitere Kanüle derart beschaffen ist, dass ein Auslass der weiteren Kanüle mit einer Schlüsselbeinarterie oder einer Leistenarterie verbindbar ist.

16. Verfahren zur Volumenentlastung eines linksseitigen Herzens, welches folgende Schritte umfasst:
a) Kanülierung des linken Atriums, wobei eine Kanüle durch den Brustkorb zum linken Atrium und ein Einlass einer Kanüle durch eine Wand des linken Atriums in das linke Atrium geführt wird;
b) Verbinden eines Nahtrings der Kanüle mit der Wand des linken Atriums;
c) Verbinden eines Auslass der Kanüle mit einem Einlass einer Herzpumpe.

17. Verfahren nach Anspruch 16, wobei die Kanüle ferner einer Druckmessleitung umfasst und ein Auslass der Druckmessleitung mit einem externen Druckmesssystem verbunden wird.

18. Verfahren nach einem der Ansprüche 16 oder 17, wobei die Kanüle über einen Führungsdraht zum linken Atrium geschoben wird.

19. Verfahren nach einem der Ansprüche 16 bis 18, wobei die Kanüle zwischen zwei Rippen in einen Interkostalraum zum linken Atrium geschoben wird, wobei vorzugsweise mindestens ein Abstandshalter zwischen den Rippen angeordnet wird, so dass ein Lumen der Kanüle durch die Rippen nicht abgeklemmbar ist.
